# EUROPEAN PATENT SPECIFICATION

(11) **EP 1 968 922 B1**
(45) Date of publication and mention of the grant of the patent: **22.04.2015**
(21) Application number: 07716229.5
(22) Date of filing: 03.01.2007
(51) Int. Cl.: C07C 17/087, C07C 21/19, C09K 5/00

(54) **METHOD FOR PRODUCING FLUORINATED ORGANIC COMPOUNDS**
VERFAHREN ZUR HERSTELLUNG FLUORINIERTER ORGANISCHER VERBINDUNGEN
PROCEDE DE FABRICATION DE COMPOSES ORGANIQUES FLUORES

(30) Priority: 03.01.2006 US 755485 P
(43) Date of publication of application: 17.09.2008
(60) Divisional application: 12178195.9
(73) Proprietor: Honeywell International Inc., Morristown, NJ 07960 (US)
(72) Inventor: TUNG, Hsueh Sung, Getzville, NY 14068 (US); MUKHOPADHYAY, Sudip, Williamsville, NY 14221 (US); VAN DER PUY, Michael, Amherst, NY 14221 (US); MA, Jing, Ji, West Seneca, NY 14224 (US); BORTZ, Cheryl, N Tonawanda, NY 14120 (US); LIGHT, Barbara, Niagara Falls, NY 14305 (US); PHILLIPS, Steven, D., Buffalo, NY 14218 (US); DUBEY, Rajesh, Buffalo, NY 14210 (US); MERKEL, Daniel C., West Seneca, NY 14224 (US)
(74) Representative: Cornish, Kristina Victoria Joy
(86) International application number: PCT/US2007/000056
(87) International publication number: WO 2007/079431

(56) References cited:
- HENNE, ALBERT L. ET AL: "Fluorinated derivatives of propane and propylene. VI" JOURNAL OF THE AMERICAN CHEMICAL SOCIETY, vol. 68, 1946, pages 496-497, XP002448570
- PALETA, OLDRICH ET AL: "Synthesis of perfluoroallyl chloride and some chlorofluoropropenes" BULLETIN DE LA SOCIETE CHIMIQUE DE FRANCE, no. 6, 1986, pages 920-924, XP009088473
- MARIA O. BURGIN ET AL: "UNIMOLECULAR REACTION KINETICS OF CF2CLCF2CH3 AND CF2CLCF2CD3: EXPERIMENTAL EVIDENCE FOR A NOVEL 1,2-FCL REARRANGEMENT PATHWAY" J. PHYS. CHEM. A, vol. 105, 2001, pages 1615-1621, XP002448571

## Description

### BACKGROUND OF INVENTION

### (1) Field of Invention:

This invention relates to novel methods for preparing fluorinated organic compounds, and more particularly to methods of producing fluorinated olefins.

### (2) Description of Related Art:

Hydrofluorocarbons (HFC's), in particular hydrofluoroalkenes such tetrafluoropropenes (including 2,3,3,3-tetrafluoro-1-propene (HFO-1234yf) and 1,3,3,3-tetrafluoro-1-propene (HFO-1234ze)) have been disclosed to be effective refrigerants, fire extinguishants, heat transfer media, propellants, foaming agents, blowing agents, gaseous dielectrics, sterilant carriers, polymerization media, particulate removal fluids, carrier fluids, buffing abrasive agents, displacement drying agents and power cycle working fluids. Unlike chlorofluorocarbons (CFCs) and hydrochlorofluorocarbons (HCFCs), both of which potentially damage the earth's ozone layer, HFCs do not contain chlorine and thus pose no threat to the ozone layer.

Several methods of preparing hydrofluoroalkenes are known. For example, U.S. Pat No. 4,900,874 (Ihara et al) describes a method of making fluorine containing olefins by contacting hydrogen gas with fluorinated alcohols. Although this appears to be a relatively high-yield process, for commercial scale production the handling of hydrogen gas at high temperature raises difficult safety related questions. Also, the cost of producing hydrogen gas, such as building an on-site hydrogen plant, can be in many situations prohibitive.

U.S. Pat. No. 2,931,840 (Marquis) describes a method of making fluorine containing olefins by pyrolysis of methyl chloride and tetrafluoroethylene or chlorodifluoromethane. This process is a relatively low yield process and a very large percentage of the organic starting material is converted in this process to unwanted and/or unimportant byproducts..

The preparation of HFO-1234yf from trifluoroacetylacetone and sulfur tetrafluoride has been described. *See* Banks, et al., Journal of Fluorine Chemistry, Vol. 82, Iss 2, p. 171-174 (1997). Also, U.S. Pat. No. 5,162,594 (Krespan) discloses a process wherein tetrafluoroethylene is reacted with another fluorinated ethylene in the liquid phase to produce a polyfluoroolefin product.

Journal of the American Chemical Society (1946) 68: 496-497 describes a study of atomic distances in polyfluorinated groups, including in propane and propene derivatives. The document describes the synthesis of a number of polyfluorinated propanes and propenes for this purpose, including a six-step process for the synthesis of CH₂=CFCF₃ (HFO-1234yf) and which starts from CH₂ClCHClCH₂Cl, and which proceeds via CH₂=CClCH₂Cl.

Bulletin de la Societe Chimique de France, EN (1986) 6: 920-924 describes the synthesis of perfluoroallylchloride, and some chlorofluoropropenes which are intermediates en route to this material.

Journal of Physical Chemistry A (2001) 105: 1615-1621 investigates the reaction kinetics of reactions involving halogenated alkanes, with a view to providing evidence for a novel rearrangement pathway. Decomposition of CF₂ClCF₂CH₃ and CF₂ClCH₂CD₃ resulted in decomposition products including CF₃CF=CH₂(HFO-1234yf).

### SUMMARY

Applicants have discovered a method for producing fluorinated organic compounds, including hydrofluoropropenes, as is defined in claim I. As used herein and throughout, unless specifically indicated otherwise, the term "converting" includes directly converting (for example, in a single reaction or under essentially one set of reaction conditions, an example of which is described hereinafter) and indirectly converting (for example, through two or more reactions or using more than a single set of reaction conditions).

Preferably the tetrachloropropene starting material is selected from the group consisting of CH₂=CClCCl₃, CCl₂=CClCH₂Cl, CHCl=CClCCl₂H, and combinations of these.

In certain preferred embodiments, the step of converting a compound of Formula (I) to HFO-1234yf comprises directly converting a compound of Formula (I). In other embodiments, the step of converting a compound of Formula (I) to HFO-1234yf comprises indirectly converting a compound of Formula (I).

An example of indirect conversion embodiments includes converting a tetrachloropropene of Formula (I)

Cl(X)₂=CCl(X)₃ (I)

where X is independently selected from H and Cl provided that the total number of Cl atoms is 4, to CF₃CCl=CH₂ (HFO-1233xf) and reacting said monochlorotrifluoropropene under conditions effective to produce CF₃CFClCH₃ (HFO-244bb), which in turn is preferably exposed to reaction conditions effective to produce HFO-1234yf. In preferred embodiments said exposing step comprises conducting one or more of said reactions in a gas phase in the presence of a catalyst, preferably a metal-based catalyst. Examples of such preferred conversion steps are disclosed more fully hereinafter.

In certain preferred embodiments the converting step comprises exposing the tetrachloropropene of Formula (I) to one or more sets of reaction conditions effective to produce HFO-1234yf. It is contemplated that in certain embodiments the exposing step comprises reacting said tetrafluoropropene of Formula (I) under conditions effective to produce a chlorofluoropropane:

CF₃CClFC(X)₃ Formula (IBB),

where all three X are H.

The preferred conversion step of the present invention is preferably carried out under conditions, including the use of one or more reactions, effective to provide a Formula **(I)** conversion of at least, 50%, more preferably at least 75%, and even more preferably at least 90%. In certain preferred embodiments the conversion is at least 95%, and more preferably at least 97%. Further in certain preferred embodiments, the step of converting the compound of Formula (I) to produce a compound of Formula (II) is conducted under conditions effective to provide a Formula (II) yield of at least 75%, more preferably at least 85%, and more preferably at least 90%. In certain preferred embodiments a yield of 95% or greater is achieved.

### DETAILED DESCRIPTION OF PREFERRED EMBODIMENTS

One beneficial aspect of the present invention is that it enables the production of desirable fluroolefins, preferably C3 fluoroolefins, using relatively high conversion and high selectivity reactions. Furthermore, the present methods in certain preferred embodiments permit the production of the desirable fluoroolefins, either directly or indirectly, from relatively attractive starting materials. For example, 2-chloro, 2,3,3,3-tetrafluoropropane is a compound that may in certain embodiments be an advantageous starting material because such products are relatively easy to handle.

Preferably the Formula (I) compound is exposed to reaction conditions effective to produce HFO-1234yf.

Although it is contemplated that the exposure step in certain embodiments may effectively be carried out in a single reaction stage and/or under a single set of reaction conditions, as mentioned above, it is preferred in many embodiments that the conversion stepcomprise a series of reaction stages or conditions. In one preferred aspect of the present invention, the conversion step comprises: (a) reacting a compound of Formula (I) in a gas and/or liquid phase reaction in the presence of at least a first catalyst to produce HFO-1233xf; (b) reacting the at least one monochloro-trifluoro-propene compound, in a gas and/or liquid phase and preferably in the presence of at least a catalyst, preferably a second catalyst which is different than the first catalyst, to produce CF₃CFClCH₃ (HFC-244bb); and (c) reacting said compound, in a gas and/or liquid phase, to produce HFO-1234yf. Each of the preferred reaction steps is described in detail below, with the headings being used for convenience but not necessarily by way of limitation.

### I. FLUORINATION OF THE TETRACHLOROPROPENE COMPOUND OF FORMULA I

One preferred reaction step in accordance with the present invention may be described by those reactions in which the tetrachloropropene compound of Formula (I) is fluorinated to produce HFO-1233xf. In certain preferred embodiments, the present converting step comprises first reacting said compound(s) by fluorinating said compound(s), preferably with HF in a gas phase, to produce HFO-1223xf. Preferably this gas phase reaction is at least partially catalyzed.

The preferred fluorination of the tetrachloropropene compound of Formula (I) is preferably carried out under conditions effective to provide a Formula (I) conversion of at least 50%, more preferably at least 75%, and even more preferably at least 90%. In certain preferred embodiments the conversion is at least 95%, and more preferably at least 97%. Further in certain preferred embodiments, the conversion of the tetrachloropropene compound of Formula (I) comprises reacting such compound under conditions effective to produce CF₃CCHCH₂ (HFO-1233xf) at a selectivity of at least 50%, more preferably at least 70%, more preferably at least 80%, and even more preferably at least 90%, with selectivities of 95% or greater being achieved in certain embodiments.

In general, it is possible that the fluorination reaction step can be carried out in the liquid phase or in the gas phase, or in a combination of gas and liquid phases, and it is contemplated that the reaction can be carried out batch wise, continuous, or a combination of these.

For embodiments in which the reaction comprises a liquid phase reaction, the reaction can be catalytic or non-catalytic. Preferably, a catalytic process is used. Lewis acid catalyst, such as metal-halide catalysts, including antimony halides, tin halides, thallium halides, iron halides, and combinations of two or more of these, are preferred in certain embodiments. Metal chlorides and metal fluorides are particularly preferred. Examples of particularly preferred catalysts of this type include SbCl₅, SbCl₃, SbF₅, SnCl₄, TiCl₄, FeCl₃ and combinations of two or more of these.

In preferred gas phase fluorination of Formula (I) compounds, preferably the reaction is at least partially a catalyzed reaction, and is preferably carried out on a continuous basis by introducing a stream containing the compound of Formula (I) into one or more reaction vessels, such as a tubular reactor. In certain preferred embodiments, the stream containing the compound of Formula (I) is preheated to a temperature of from 80°C to 400°C, more preferably from 150°C to 400°C, and in certain embodiments preferably about 300°C, and introduced into a reaction, vessel (preferably a tube reactor), which is maintained at the desired temperature, preferably from 80°C to 700°C, more preferably from 90°C to 600°C, even more preferably in certain embodiments from 400°C to 600°C, more preferably from 450°C to 600°C, where it is preferably contacted with catalyst and fluorinating agent, such as HF.

Preferably the vessel is comprised of materials which are resistant to corrosion as Hastelloy, Inconel, Monel and/or fluoropolymers linings.

Preferably the vessel contains catalyst, for example a fixed or fluid catalyst bed, packed with a suitable fluorination catalyst, with suitable means to ensure that the reaction mixture is maintained with the desired reaction temperature range.

Thus, it is contemplated that the fluorination reaction step may be preformed using a wide variety of process parameters and process conditions in view of the overall teachings contained herein. However, it is preferred in certain embodiments that this reaction step comprise a gas phase reaction, preferably in the presence of catalyst, and even more preferably a chromium-based catalyst (such as Cr₂O₃ catalyst), an iron-based catalyst (such as FeCl₃ on carbon (designated herein as FeCl₃/C for convenience), and combinations of these. In preferred embodiments, the catalyst is a combination of the two aforementioned catalysts, where the reaction vessel contains in a first zone the chromium-based catalyst and in a second zone the iron-based catalyst. The temperature of the reaction in the chromium-based catalyst reaction is preferably kept at a temperature of from 200°C to 600°C and even more preferably from 250°C to 500°C. The temperature of the reaction in the iron-based catalyst reaction zone is preferably kept at a temperature of from 80°C to 300°C and even more preferably from 100°C to 250°C.

In general it is also contemplated that a wide variety of reaction pressures may be used for the fluorination reaction, depending again on relevant factors such as the specific catalyst being used and the most desired reaction product. The reaction pressure can be, for example, superatmospheric, atmospheric or under vacuum, and in certain preferred embodiments is from 6.9 to 1379 kPa (1 to 200 psia), and in certain embodiments from 6.9 to 827.4 kPa (1 to 120 psia).

In certain embodiments, an inert diluent gas, such as nitrogen, may be used in combination with the other reactor feed(s).

It is contemplated that the amount of catalyst use will vary depending on the particular parameters present in each embodiment.

### II. FLUORINATION OF HFO-1233xf

HFO-1233xf preferably produced as described above, then is preferably subject to further fluorination reaction(s) to produce HCFC-244. Preferably this gas phase reaction is at least partially catalyzed.

The fluorination of HFO-1233xf is preferably carried out under conditions effective to provide a HFO-1233xf conversion of at least 40%, more preferably at least 50%, and even more preferably at least 60%. Further in certain preferred embodiments, the conversion of HFO-1233xf comprises reacting such compound under conditions effective to HCFC-244, at a selectivity of at least 70%, more preferably at least 80%, and even more preferably at least 85%, with selectivities of 90% or greater being achieved in certain embodiments.

In general, it is possible that this fluorination reaction step can be carried out in the liquid phase or in the gas phase, or in a combination of gas and liquid phases, and it is contemplated that the reaction can be carried out batch wise, continuous, or a combination of these.

For embodiments in which the reaction comprises a liquid phase reaction, the reaction can be catalytic or non-catalytic. Preferably, a catalytic process is used. Lewis acid catalyst, such as metal-halide catalysts, including antimony halides, tin halides, thallium halides, iron halides, and combinations of two or more of these, are preferred in certain embodiments. Metal chlorides and metal fluorides are particularly preferred. Examples of particularly preferred catalysts of this type include SbCl₅, SbCl₃, SbF₅, SnCl₄, TiCl₄, FeCl₃ and combinations of two or more of these.

In preferred gas phase fluorination of HFO-1233xf, the reaction is at least partially a catalyzed reaction, and is preferably carried out on a continuous basis by introducing a stream containing HFO-1233xf into one or more reaction vessels, such as a tubular reactor. In certain preferred embodiments, the stream containing HFO-1233xf, is preheated to a temperature of from 50°C to 400°C, and in certain embodiments preferably about 80°C. In other embodiments, it is preferred that the stream containing HFO-1233xf, is preheated to a temperature of from 150°C to 400°C, preferably about 300°C. This steam, preferably after preheating, is then preferably introduced into a reaction vessel (preferably a tube reactor), which is maintained at the desired temperature, preferably from 50°C to 250°C, more preferably from 50°C to 150°C, where it is preferably contacted with catalyst and fluorinating agent, such as HF.

Preferably the vessel is comprised of materials which are resistant to corrosion as Hastelloy, Inconel, Monel and/or fluoropolymers linings.

Preferably the vessel contains catalyst, for example a fixed or fluid catalyst bed, packed with a suitable fluorination catalyst, with suitable means to ensure that the reaction mixture is maintained within about the desired reaction temperature range.

Thus, it is contemplated that the fluorination reaction step may be preformed using a wide variety of process parameters and process conditions in view of the overall teachings contained herein. However, it is preferred in certain embodiments that this reaction step comprise a gas phase reaction, preferably in the presence of catalyst, and even more preferably an Sb-based catalyst, such as catalyst which is about 50wt% SbCl₅/C. Other catalysts which may be used include: from 3 to 6 wt% FeCl₃/C; SbF₅/C; about 20 wt% SnCl₄/C; about 23 wt% TiCl₄/C; and activated carbon. Preferably the catalyst comprises Cl₂ and HF pre-treated SbCl₅/C.

In general it is also contemplated that a wide variety of reaction pressures may be used for the fluorination reaction, depending again on relevant factors such as the specific catalyst being used and the most desired reaction product. The reaction pressure can be, for example, superatmospheric, atmospheric or under vacuum and in certain preferred embodiments is from 6.9 to 1379 kPa (1 to 200 psia), more preferably in certain embodiments from 6.9 to 827.4 kPa (1 to 120 psia).

In certain embodiments, an inert diluent gas, such as nitrogen, may be used in combination with the other reactor feed(s).

It is contemplated that the amount of catalyst use will vary depending on the particular parameters present in each embodiment.

### III. DEHYDROHALOGENATION OF HFC-244bb

One preferred reaction step in accordance with the present invention may be described by those reactions in which HFC-244bb is dehydrohalogenated to produce HFO-1234yf. In certain preferred embodiments, the stream containing HFC-244bb is preheated to a temperature of from 150°C to 400°C, preferably about 350°C, and introduced into a reaction vessel, which is maintained at about the desired temperature, preferably from 200°C to 700°C, more preferably from 300°C to 700°C, more preferably from 300°C to 450°C**,** and more preferably in certain embodiments from 350°C to 450°C.

Preferably the vessel is comprised of materials which are resistant to corrosion as Hastelloy, Inconel, Monel and/or fluoropolymers linings. Preferably the vessel contains catalyst, for example a fixed or fluid catalyst bed, packed with a suitable dehydrohalogenation catalyst, with suitable means to heat the reaction mixture to about the desired reaction temperature.

Thus, it is contemplated that the dehydrohalogenation reaction step may be preformed using a wide variety of process parameters and process conditions in view of the overall teachings contained herein. However, it is preferred in certain embodiments that this reaction step comprise a gas phase reaction, preferably in the presence of catalyst, and even more preferably a carbon- and/or metal-based catalyst, preferably activated carbon, a nickel-based catalyst (such as Ni-mesh) and combinations of these. Other catalysts and catalyst supports may be used, including palladium on carbon, palladium-based catalyst (including palladium on aluminum oxides), and it is expected that many other catalysts may be used depending on the requirements of particular embodiments in view of the teachings contained herein. Of course, two or more any of these catalysts, or other catalysts not named here, may be used in combination.

The gas phase dehydrohalogenation reaction may be conducted, for example, by introducing a gaseous form of HFC-244bb into a suitable reaction vessel or reactor. Preferably the vessel is comprised of materials which are resistant to corrosion as Hastelloy, Inconel, Monel and/or fluoropolymers linings. Preferably the vessel contains catalyst, for example a fixed or fluid catalyst bed, packed with a suitable dehydrohalogenation catalyst, with suitable means to heat the reaction mixture to about the desired reaction temperature.

While it is contemplated that a wide variety of reaction temperatures may be used, depending on relevant factors such as the catalyst being used and the most desired reaction product, it is generally preferred that the reaction temperature for the dehydrohalogentation step is from 200°C to 800°C**,** more preferably from 400°C to 800°C, and even more preferably from 400°C to 500°C, and more preferably in certain embodiments from 300°C to 500°C.

In general it is also contemplated that a wide variety of reaction pressures may be used, depending again on relevant factors such as the specific catalyst being used and the most desired reaction product. The reaction pressure can be, for example, superatmospheric, atmospheric or under vacuum, and in certain preferred embodiments is from 6.9 to 1379 kPa (1 to 200 psia), and even more preferably in certain embodiments from 6.9 to 827.4 kPa (1 to 120 psia).

In certain embodiments, an inert diluent gas, such as nitrogen, may be used in combination with the other reactor feed(s). When such a diluent is used, it is generally preferred that the compound of Formula (I), preferably HIFC-244bb, comprise from 50% to greater than 99% by weight based on the combined weight of diluent and Formula (I) compound.

It is contemplated that the amount of catalyst use will vary depending on the particular parameters present in each embodiment.

Preferably in such dehydrofluorination embodiments as described in this section, the conversion of HFC-244bb is at least 60% more preferably at least 75%, and even more preferably at least 90%. Preferably in such embodiments, the selectivity to compound of Formula (II), preferably HFO-1234yf, is at least 50%, more preferably at least 70% and more preferably at least 80%.

### EXAMPLES

Additional features of the present invention are provided in the following examples, which should not be construed as limiting the claims in any way.

### Example 1

### Preparation of CCl₂=CClCH₂Cl from HCCl₂CCl₂CH₂Cl

Aliquat-336® (about 0.26 g) and about 24.8 g of HCCl₂CCl₂CH₂Cl were stirred rapidly at room temperature while adding about 20 g of 25 % aqueous NaOH over 19 minutes. Stirring was continued overnight before adding 30 mL water and allowing the phases to separate. The lower organic phase, in an amount of about 19.8 g, was about 97.5 % pure CCl₂=CClCH₂Clby GC analysis (96 % yield). Prior to fluorination, it was distilled (bp 69 to 72 °C at about 30 mm Hg) to remove any phase transfer catalyst. H NMR: δ 4.41 (s) ppm.

### Example 2

### Selective catalyzed-transformation of CCl₂=CClCH₂Cl to CF₃CCl=CH₂ (HFO-1233xf) in gas-phase

A 55.9 cm (22-inch) long and 1.3 cm (1/2-inch) diameter Monel pipe gas-phase reactor is charged with about 120 cc of a catalyst or a mixture of two catalysts. In case of a mixture, Cr₂O₃ catalyst is kept at the bottom zone of the reactor at a constant temperature of about 270°C-500°C and the other catalyst, such as FeCl₃/C, is kept at the middle and the top zone of the reactor at a constant temperature of about 120°C - 220°C. The reactor is mounted inside a heater with three zones (top, middle, and bottom). The reactor temperature is read by custom-made-5-point thermocouples kept inside at the middle of the reactor. The bottom of the reactor is connected to a pre-heater, which is kept at 300°C by electrical heating. The liquid-HF is fed from a cylinder into the preheater through a needle valve, liquid mass-flow meter, and a research control valve at a constant flow of 1 to 1000 grams pre hour (g/h). The HF cylinder is kept at a constant pressure of 411.6 kPa (45 psig) by applying anhydrous N₂ gas pressure into the cylinder head space. 10 to. 1000 g/h of CCl₂=CClCH₂Cl is fed as a liquid through a dip tube from a cylinder under about 411.6 kPa (45 psig) of N₂ pressure. The organic flows from the dip tube to the preheater (kept at about 250°C) through a needle valve, liquid mass-flow meter, and a research control valve at a constant flow of 1-1000 g/h. The organic is also fed as a gas while heating the cylinder containing organic at about 220°C. The gas coming out of the cylinder is passed through a needle valve and a mass flow controller into the preheater. The organic line from the cylinder to the pre-heater is kept at about 200°C by wrapping with constant temperature heat trace and electrical heating elements. All feed cylinders are mounted on scales to monitor their weight by difference. The catalysts are dried at the reaction temperature over a period of about 8 hours and then pretreated with about 50 g/h of HF under atmospheric pressure over a period of about 6 hours and then under 446.1 kPa (50 psig) HF pressure over another period of about 6 hours before contacting with organic feed containing CCl₂=CClCH₂Cl. The reactions are run at a constant reactor pressure of 101.3 to 1135.5 kPa (0 to 150 psig) by controlling the flow of reactor exit gases by another research control valve. The gases exiting reactor are analyzed by on-line GC and GC/MS connected through a hotbox valve arrangement to prevent condensation. The conversion of CCl₂=CClCH₂Cl is 70 to 100% and the selectivity to 1233xf is. 80% to 95%, respectively. The product is collected by flowing the reactor exit gases through a scrubber solution comprising 20wt% to 60 wt%. KOH in water and then trapping the exit gases from the scrubber into a cylinder kept in dry ice or liquid N₂. The product, 1233xf is then substantially isolated by distillation. The results are tabulated in Table 1.

**Table 1: Transformation of CCl₂=CClCH₂Cl to CF₃CCl=CH₂ (CCl₂=CClCH₂Cl + 3HF → CF₃CCl=CH₂ + 3HCl)**

| **#** | **Catalyst** | **T, °C** | **HF flow, g/b** | **CCl₂=CClCH₂Cl flow, g/h** | **% Conv of CCl₂=CClCH₂Cl** | **% Sel to 1233xf** |
|---|---|---|---|---|---|---|
| **1** | 10% v/v Cr₂O₃-90% v/v FeCl₃/C | 350/150 | 50 | 12 | 79 | 81 |
| **2** | 20% v/v Cr₂O₃-80% v/v FeCl₃/C | 350/150 | 50 | 12 | 83 | 86 |
| **3** | 30% v/v Cr₂O₃-70% v/v FeCl₃/C | 350/150 | 50 | 12 | 89 | 96 |
| **4** | 30% v/v Cr₂O₃-70% v/v FeCl₃/C | 350/150 | 70 | 12 | 79 | 93 |
| **5** | 30% v/v Cr₂O₃-70% v/v FcCl₃/C | 345/170 | 50 | 25 | 85 | 90 |
| **6** | Cr₂O₃ | 350 | 50 | 20 | 90 | 93 |
| **7** | FeCl₃/C | 150 | **50** | **20** | **74** | **39** |
| **8** | SbCl₅/C | 150 | **50** | **20** | **81** | **52** |

| | | | | | | |
|---|---|---|---|---|---|---|
| Reaction conditions: Catalyst used (total) 120 cc; pressure, 111.7 kPa (1.5 psig); | | | | | | |

### Examples 3A and 3B

### Liquid-phase catalytic fluorination of CF₃CCl=CH₂ (1233xf) with HF to CF₃CFClCH₃ (244bb)

### Example 3A

About 327 grams of HF, about 50 grams 1233xf, and about 75 grams SbCl₅ were charged into a 1-L autoclave. The reaction mixture was stirred at a temperature of about 80°C for about 3 hours under about 4376.1 kPa (620 psig) of pressure. After the reaction, the reactor was cooled to about 0°C and about 300 ml water was then added slowly into the autoclave over a period of about 45 min. After complete addition of water under stirring, the reactor was cooled to room temperature and then the overhead gases were transferred to another collecting cylinder. The yield of CF₃CFClCH₃ was about 90% at a 1233xf conversion level of about 98%. The other major by-products were CF₃CF₂CH₃ (2%), and an unidentified isomer of a C4 compound of the general formula, C₄H₃Cl₃F₄ (8%).

### Example 3B

About 327 grams HF, about 50 grams 1233xf, and about 75 grams SbCl₅ were charged into a 1-L autoclave. The reaction mixture was stirred at 80°C for about 3 hours under about 4410.5 kPa (625 psig) of pressure. After the reaction, the reactor was cooled to about 45°C and then the overhead gas mixture was passed through a well dried KF, NaF, or Al₂O₃ (350 g) packed column kept at about 80°C to strip off HF from the gas stream. The gases coming out of the column are collected in a cylinder kept in dry ice (-70°C) bath. The yield of CF₃CFClCH₃ was 87% at a 1233xf conversion level of 93%. The other major by-products were CF₃CF₂CH₃ (1 %), and an unidentified isomer of a C4 compound of the general formula, C₄H₃Cl₃F₄ (7%). The product, CF₃CFClCH₃ was isolated by distillation with 98% purity.

### Example 4

### Gas-phase catalytic fluorination of CF₃CCl=CH₂ (1233xf) with HF to CF₃CFClCH₃ (244bb)

A 55.9 cm (1.3 cm diameter) [22-inch (1/2-inch diameter)] Monel tube gas phase reactor was charged with about 120 cc of a catalyst. The reactor was mounted inside a heater with three zones (top, middle and bottom). The reactor temperature was read by a custom made 5-point thermocouple kept at the middle inside of the reactor. The inlet of the reactor was connected to a pre-heater, which was kept at about 300°C by electrical heating. Organic (1233xf) was fed from a cylinder kept at 70°C through a regulator, needle valve, and a gas mass-flow-meter. The organic line to the pre-heater was heat traced and kept at a constant temperature of about 73°C by electrical heating to avoid condensation. N₂ was used as a diluent in some cases and fed from a cylinder through a regulator and a mass flow controller into the pre-heater. All feed cylinders were mounted on scales to monitor their weight by difference. The reactions were run at a constant reactor pressure of from 101.3 to 790.8 kPa (0 to 100 psig) by controlling the flow of reactor exit gases by another research control valve. The gas mixtures exiting reactor was analyzed by on-line GC and GC/MS connected through a hotbox valve arrangements to prevent condensation. The conversion of 1233xf was from 50% to 65% and the selectivity to 244 isomer (CF₃CFClCH₃) was from 90% to

93% depending on the reaction conditions using 120 co of 50 wt% SbCl₅/C as the catalyst at 65°C to -85°C with a HF flow of about 50 g/h and organic flow of about 15 g/h. No CF₃CF₂CH₃ was observed under the reaction conditions. The catalyst is pretreated at first with 50 g/h HF at about 65°C for about 2 hours and then with about 50 g/h HF and about 200 seem of Cl₂ at about 65°C for about 4 hours. After pre-treatment, about 50 sccm of N₂ is flows over a period of about 40 minutes through the catalyst bed to sweep free chlorine from the catalyst surface prior to interacting with the organic feed (1233xf). Pretreatment is considered important to many embodiments of the invention. The products were collected by flowing the reactor exit gases through a 20-60 wt% aqueous KOH scrubber solution and then trapping the exit gases from the scrubber into a cylinder kept in dry ice or liquid N₂. The products were then isolated by distillation. About 50 wt% SbCl₅/C, 3 to 6 wt% FeCl₃/C, 20 wt% SnCl₄/C, and about 23 wt% TiCl₄/C, using 4 different kind of activated carbon such as Shiro saga, Calgon, Norit, and Aldrich were used as the catalyst at from about 60 to about 150°C. Among all the catalysts used for this reaction, Cl₂ and HF pretreated SbCl₅/C was found to be generally preferred in terms of activity. The results using SbCl₅ as the catalyst are shown in Table 2.

**Table 2: Catalyzed-gas-phase transformation of CF₃CCl=CH₂ to CF₃CFClCH₃**

| # | Cat | T, °C | Conv. of CF₃CCl=CH₂ (1233xf) | Sel. to CF₃CFClCH₃ |
|---|---|---|---|---|
| 1 | 10 wt% SbCl₅/C | 60 | 15 | 100 |
| 2 | 20 wt% SbCl₅/C | 60 | 21 | 98 |
| 3 | 30 wt% SbCl₅/C | 60 | 32 | 98 |
| 4 | 50 wt% SbCl₅/C | 60 | 55 | 97 |
| 5 | 50 wt% SbCl₅/C | 80 | 62 | 93 |
| 6 | 50 wt% SbCl₅/C | 100 | 56 | 87 |
| 7 | 60 wt% SbCl₅/C | 60 | 59 | 91 |
| 8 | 50 wt% SbCl₅/NORIT RFC 3 Activated Carbon | 60 | 34 | 92 |
| 9 | 50 wt% SbCl₅/Shiro Saga Activated Carbon | 60 | 56 | 96 |
| 10 | 50 wt% SbCl₅/Aldrich Activated Carbon | 60 | 57 | 94 |

**Reaction conditions:** 1233xf flow, 150 sccm; HF flow 50g/h; pressure 118.6 to 137.9 kPa (2.5-5.3 psig); in 1-5 reactions Calgon activated carbon is used as the catalyst support; catalyst, 120 cc. All catalysts are pre-treated with Cl₂ and HF prior to contacting with 1233xf.

### Example 5

### Conversion of CF₃CFClCH₃ to CF₃CF=CH₂ in gas-phase

A 55.9 cm (1.3 diameter) [22 inch (1/2 inch diameter)] Monel tube gas phase reactor was charged with 120 cc of catalyst. The reactor was mounted inside a heater with three zones (top, middle and bottom). The reactor temperature was read by custom made 5-point thermocouples kept at the middle inside of the reactor. The inlet of the reactor was connected to a pre-heater, which was kept at about 300°C by electrical heating. Organic (CF₃CFClCH₃) was fed from a cylinder kept at about 65°C through a regulator, needle valve, and a gas mass-flow-meter. The organic line to the pre-heater was heat traced and kept at a constant temperature of from about 65°C to about 70°C by electrical heating to avoid condensation. The feed cylinder was mounted on scales to monitor their weight by difference. The reactions were run at a constant reactor pressure of from 101.3 to 790.8 kPa (0 to 100 psig) by controlling the flow of reactor exit gas by another research control valve. The gas mixture exiting reactor was analyzed by on-line GC and GC/MS connected through a hotbox valve arrangement to prevent condensation. The conversion of CF₃CFClCH₃ was almost 98% and the selectivity to HFO-1234yf was from 69% to 86% depending on the reaction conditions. The products were collected by flowing the reactor exit gases through a 20wt% to 60 wt% of aquesous KOH scrubber solution and then trapping the exit gases from the scrubber into a cylinder kept in dry ice or liquid N₂. The products were then isolated by distillation. Results are tabulated in T-able 3.

**Table 3: Catalyzed-transformation of CF₃CFClCH₃ to HFO-1234yf**

| # | Cat | T, °C | | Flow rate, CF₃CFClCH₃ (244 isomer) sccm | Conversion of 244 | 1234yf (Sel. %) |
|---|---|---|---|---|---|---|
| 1 | A | 400 | | 150 | 100 | 46 |
| 2 | B | 400 | | 150 | 96 | 63 |
| 3 | C | 400 | | 100 | 100 | 64 |
| 4 | D | 400 | | 100 | 99 | 93 |
| 5 | D | 400 | | 150 | 92 | 89 |

| # | Cat | T, °C | | Flow rate, CF₃CFClCH₃ (244 isomer) seem | Conversion of 244 | 1234yf (Sel. %) |
|---|---|---|---|---|---|---|
| 6 | E | 400 | | 100 | 96 | 56 |
| 7 | F | 400 | | 100 | 87 | 51 |
| 8 | G | 400 | | 100 | 100 | 37 |

**Reaction** conditions: pressure, 118.6 to 137.9 kPa (2.5-5.3 psig); catalyst, 100 cc, A is NORIT RFC 3; B is Shiro-Saga activated carbon, C is Aldrich activated carbon; D is Calgon activated carbon; activated carbon; E is 0.5 wt% Pd/C; F is 0.5 wt% Pt/C; G is Ni-mesh; Organic cylinder temperature -65°C; CF₃CFClCH₃ (244) line to the preheater-60°C; Preheater, 350°C; P135.8 kPa (5 psig).

### Example 6

### Selective catalyzed-transformation of CCl₃CCl=CH₂ to CF₃CCl=CH₂ (HFO-1233xf) in gas-phase

A 55.9 cm (22-inch) long and 1.3 cm (1/2-inch) diameter Monel pipe gas phase reactor was charged with 120 cc of a catalyst or a mixture of two catalysts. In case of a mixture, Cr₂O₃ catalyst is kept at the bottom zone of the reactor at a substantially constant temperature of from 270°C to 500°C and the other catalyst, such as FeCl₃/C is kept at the middle and the top zone of the reactor at a substantially constant temperature of from 120°C to 220°C. The reactor was mounted inside a heater with three zones (top, middle, and bottom). The reactor temperature was read by custom-made-5-point thermocouples kept inside at the middle of the reactor. The bottom of the reactor was connected to a pre-heater, which was kept at about 300°C by electrical heating. The liquid-HF was fed from a cylinder into the pre-heater through a needle valve, liquid mass-flow meter, and a research control valve at a substantially constant flow of from 1 to 1000 g/h. The HF cylinder was kept at a substantially constant pressure of about 411.6 kPa (45 psig) by applying anhydrous N₂ gas pressure into the cylinder head space. A feed rate of from about 10 g/h to about 1000 g/h of CCl₃CCl=CH₂ was fed as a liquid through a dig tube from a cylinder under about 411.6 kPa (45 psig)of N₂ pressure. The organic was flown from the dip tube to the pre-heater (kept at about 250°C) through needle valve, liquid mass-flow meter, and a research control valve at a substantially constant flow of from about 1 to about 1000 g/h. The organic is also fed as a gas while heating the cylinder containing organic at about 220°C. The gas effluent from the cylinder is passed through a needle valve and a mass flow controller into the pre-heater. The organic line from the cylinder to the pre-heater was kept at about 200°C by wrapping with constant temperature heat trace and electrical heating elements. All feed cylinders were mounted on scales to monitor their weight by difference. The catalysts were dried at the reaction temperature over a period of about 8 hours and then pretreated with about 50 g/h of HF under atmospheric pressure over a 6 hour period and then under about 446.1 kPa (50 psig) HF Pressure over a 6 hour period before contacting with organic feed, CCl₃CCl=CH₂. The reactions were run at a substantially constant reactor pressure ranging from 101.3 to 1135.5 kPa (0 to 150 psig) by controlling the flow of reactor exit gases by another research control valve. Those gases exiting reactor were analyzed by on-line GC and GC/MS connected through a hotbox valve arrangements to prevent condensation. The conversion of CCl₃CCl=CH₂ was in a range of from 90% to 100% and the selectivity to CF₃CCl=CH₂ (1233xf) was about 79%. The effluent contained in addition HFO-1243zf in an amount of about 7.7%, 1232-isomer in an amount of about 1.3%, and 1223 in an amount of about 0.8%, and an unidentified byproduct. The product was collected by flowing the reactor exit gases through a 20-60 wt% aq. KOH scrubber solution and then trapping the exit gases from the scrubber into a cylinder kept in dry ice or liquid N₂. The product, 1233xf was then substantially isolated by distillation. Using only Cr₂O₃ catalyst, a selectivity of about 68% to 1233xfat a conversion level of about 79% was achieved.

### Examples 7A - 7D

### Direct Liquid-phase catalytic fluorination of CCl₃CCl=CH₂ with HF to CF₃CFClCH₃ (244-isomer)

### Example 7A

About 327 grams HF, about 50 grams CCl₃CCl=CH₂, and about 75 grams SbCl₅ were charged into a 1-L autoclave. The reaction mixture was stirred at about 80°C for about 3 hours under about 4307.1 kPa (610 psig) of pressure. After the reaction, the reactor was cooled to about40°C and about 300 ml water was then added slowly into the autoclave over a period of about 45 min. After complete addition of water under stirring, the reactor was cooled to about room temperature and then the overhead gases were transferred to another collecting cylinder. The yield of CF₃CFClCH₃ was about 89% at a CCl₃CCl=CH₂ conversion level of about 88%. The other major by-products were CF₃CF₂CH₃ (2%), and an unidentified isomer of a C4 compound of the general formula, C₄H₃Cl₃F₄ (8%).

### Example 7B

About 327 grams HF, about 50 grams CCl₃CCl=CH₂, and about 75 grams SbCl₅ were charged into a 1-L autoclave. The reaction mixture was stirred at about 100°C for about 3 hours under about 4824.2 kPa (685 psig) of pressure. After the reaction, the reactor was cooled to about 40°C and about 300 ml water was then added slowly into the autoclave over a period of about 45 minutes. After complete addition of water. under stirring, the reactor was cooled to room temperature and then the overhead gases were transferred to another collecting cylinder. The yield of CF₃CFClCH₃ was about 78% at a CCl₃CCl=CH₂ conversion level of about 100%. The other major by-products were CF₃CF₂CH₃ (about 4%), and an unidentified isomer of a C4 compound of the general formula, C₄H₃Cl₃F₄ (about 13%).

### Example 7C

About 327 grams HF, about 50 grams CCl₃CCl=CH₂, and about 75 grams SbCl5 were charged into a 1-L autoclave. The reaction mixture was stirred at about 125°C for about 6 hours under about 5789.5 kPa (825 psig) of pressure. After the reaction, the reactor was cooled to about 40°C and about 300 ml water was then added slowly into the autoclave over a period of about 45 min. After complete addition of water under stirring, the reactor was cooled to about room temperature and then the overhead gases were transferred to another collecting cylinder. The major products were CF₃CF₂CH₃ (about 53%) and CF₃CFClCH₃ (about 25%) at a CCl₃CCl=CH₂ conversion level of about 100%. The other major by-products were and unidentified isomer of a C4 compound of the general formula, C₄H₃Cl₃F₄ (8%) and tar.

### Example 7D

About 327 grams HF, about 50 grams CCl₃CCl=CH₂, and about 75 g SbCl₅ were charged into a 1-L autoclave. The reaction mixture was stirred at about 150°C for at about 6 hours under about 5789.5 kPa (825 psig) of pressure. After the reaction, the reactor was cooled to about 40°C and about 300 ml water was then added slowly into the autoclave over a period of about 45 minutes. After complete addition of water under stirring, the reactor was cooled to about room temperature and then the overhead gases were transferred to another collecting cylinder. The major products were CF₃CF₂CH₃ (about 57%) and CF₃CFClCH₃ (about 15%) at a CCl₃CCl=CH₂ conversion level of about 100%. The other major by-products were and unidentified isomer of a C4 compound of the general formula, C₄H₃Cl₃F₄ (about 11 %) and tar.

### Example 8

### Catalytic conversion of CF₃CF₂CH₃ to CF₃CF=CH₂

A 55.9 cm (1.3 diameter) [22 inch (1/2 inch diameter)] Monel tube gas phase reactor was charged with 120 cc of a catalyst. The reactor was mounted inside a heater with three zones (top, middle and bottom). The reactor temperature was read by custom made 5-point thermocouples kept at the middle inside of the reactor. The inlet of the reactor was connected to a pre-heater, which was kept at about 300°C by electrical heating. Organic material (245cb) was fed from a cylinder kept at about 65°C through a regulator, needle valve, and a gas mass-flow-meter. The organic line to the pre-heater was heat traced and kept at a substantially constant temperature in a range of from

65°C to 70°C by electrical heating to avoid condensation. The feed cylinder was mounted on a scale to monitor its weight by difference. The reactions were run at a substantially constant reactor pressure of from 101.3 to 790.8 kPa (0 to 100 psig) by controlling the flow of reactor exit gases by another research control valve. The gas mixtures exiting reactor was analyzed by on-line GC and GC/MS connected through a hotbox valve arrangements to prevent condensation. The conversion of 245cb was in the range of from 30% to 70% and the selectivity to 1234yf was in the range of from 90% to 100% depending on the reaction conditions. The products were collected by flowing the reactor exit gases through a 20-60-wt% of aq. KOH scrubber solution and then trapping the exit gases from the scrubber into a cylinder kept in dry ice or liquid N₂. The products were then substantially isolated by distillation. Results are tabulated in Table 4.

**Table 4: Transformation of CF₃CF₂CH₃ to 1234yf**

| # | Cat | T, °C | H₂, sccm | CF₃CF₂CH₃ (245cb) seem | Conversion of 245cb, % | 1234yf (Sel. %) |
|---|---|---|---|---|---|---|
| 1 | A | 575 | 0 | 65 | 79 | 63 |
| 2 | B | 575 | 0 | 68 | 82 | 57 |
| 3 | C | 575 | 0 | 73 | 73 | 61 |
| 4 | D | 575 | 0 | 68 | 84 | 59 |
| 5 | D | 575 | 20 | 68 | 89 | 73 |
| 6 | E | 550 | 0 | 69 | 92 | 53 |
| 7 | F | 550 | 0 | 67 | 93 | 33 |
| 8 | G | 550 | 0 | 69 | 73 | 46 |

**Reaction conditions:** pressure, 118.6 to 137.9 kPa (2.5-5.3 psig); catalyst, 100 cc, A is NORIT RFC 3; B is Shiro-Saga activated carbon; C is Aldrich activated carbon; D is Calgon activated carbon; E is 0.5 wt% Pd/C; F is 0.5 wt% Pt/C; G is Ni-mesh; Organic cylinder temperature is about 65°C; CF₃CF₂CH₃ (245cb) line to the preheater is maintained at about 50°C; preheater temperature is maintained at about 350°C; N₂ flow is not used; pressure is maintained at about 122.0 kPa (3 psig).

## Claims

1. A method for producing 2,3,3,3-tetrafluoro-1-propene (HFO-1234yf), comprising;
i) fluorinating a tetrachloropropene of Formula (I)
CX₂=CClCX₃ (I)
where X is independently selected from H and Cl provided that the total number of Cl atoms is 4, to form CF₃CCl=CH₂ (HFO-1233xf);
ii) fluorinating HFO-1233xf to form CF₃CFClCH₃ (HFC-244bb); and
iii) subjecting HFC-244bb to a dehydrohalogenation reaction, to form HFO-1234yf.

2. A method according to claim 1, wherein said tetrachloropropene of Formula (I) is selected from the group consisting of CH₂=CClCCl₃, CCl₂=CClCH₂Cl, CHCl=CClCCl₂H, and combinations of these.

3. A method according to claim 1 or claim 2, wherein said dehydrohalogenation comprises at least one gas phase catalytic reaction.

4. A method according to any preceding claim, wherein fluorinating the tetrachloropropene of Formula (I) is conducted using hydrogen fluoride in the gas phase.

5. A method according to claim 4, wherein fluorinating the tetrachloropropene of Formula (I) is conducted in the presence of a catalyst.

6. A method according to any preceding claim, wherein fluorinating HFO-1233xf is conducted in the gas phase.

7. A method according to any of claims 1 to 5, wherein fluorinating HFO-1233xf is conducted in the liquid phase.

8. A method according to claim 6 or claim 7, wherein fluorinating HFO-1233xf is conducted in the presence of a catalyst.

9. A method according to any preceding claim, wherein the dehydrohalogenation reaction is conducted at a temperature in the range of 200 °C to 800 °C.

10. A method according to any preceding claim, wherein the tetrachloropropene of Formula (I) comprises CCl₂=CClCH₂Cl.

11. A method according to any of claims 1 to 9, wherein the tetrachloropropene of Formula (I) comprises CCl₃CCl=CH₂.

## Patentansprüche

1. Verfahren zur Herstellung von 2,3,3,3-Tetrafluor-1-propen (HFO-1234yf), bei dem man:
i) ein Tetrachlorpropen der Formel (I)
CX₂=CClX₃ (I)
wobei X unabhängig aus H und Cl ausgewählt ist, mit der Maßgabe, dass die Gesamtzahl von Cl-Atomen 4 beträgt, zu CF₃CCl=CH₂ (HFO-1233xf) fluoriert;
ii) HFO-1233xf zu CF₃CFClCH₃ (H-FKW 244bb) fluoriert und
iii) H-FKW 244bb einer Dehydrohalogenierungsreaktion zur Bildung von HFO-1234yf unterwirft.

2. Verfahren nach Anspruch 1, bei dem man das Tetrachlorpropen der Formel (I) aus der Gruppe bestehend aus CH₂=CClCCl₃, CCl₂=CClCH₂Cl, CHCl=CClCl₂H und Kombinationen davon auswählt.

3. Verfahren nach Anspruch 1 oder Anspruch 2, bei dem die Dehydrohalogenierung mindestens eine katalytische Reaktion in der Gasphase umfasst.

4. Verfahren nach einem der vorhergehenden Ansprüche, bei dem man die Fluorierung des Tetrachlorpropens der Formel (I) mit Fluorwasserstoff in der Gasphase durchführt.

5. Verfahren nach Anspruch 4, bei dem man die Fluorierung des Tetrachlorpropens der Formel (I) in Gegenwart eines Katalysators durchführt.

6. Verfahren nach einem der vorhergehenden Ansprüche, bei dem man die Fluorierung von HFO-1233xf in der Gasphase durchführt.

7. Verfahren nach einem der Ansprüche 1 bis 5, bei dem man die Fluorierung von HFO-1233xf in der Flüssigphase durchführt.

8. Verfahren nach Anspruch 6 oder Anspruch 7, bei dem man die Fluorierung von HFO-1233xf in Gegenwart eines Katalysators durchführt.

9. Verfahren nach einem der vorhergehenden Ansprüche, bei dem man die Dehydrohalogenierungsreaktion bei einer Temperatur im Bereich von 200°C bis 800°C durchführt.

10. Verfahren nach einem der vorhergehenden Ansprüche, bei dem das Tetrachlorpropen der Formel (I) CCl₂=CClCH₂Cl umfasst.

11. Verfahren nach einem der Ansprüche 1 bis 9, bei dem das Tetrachlorpropen der Formel (I) CCl₃CCl=CH₂ umfasst.

## Revendications

1. Méthode de production de 2,3,3,3-tétrafluoro-1-propène (HFO-1234yf), comprenant :
i) la fluoration d'un tétrachloropropène de Formule (I)
CX₂=CClCX₃ (I)
dans laquelle X est choisi indépendamment parmi H et Cl, à condition que le nombre total d'atomes de Cl soit de 4, pour former CF₃CCl=CH₂ (HFO-1233xf) ;
ii) la fluoration de HFO-1233xf pour former CF₃CFClCH₃ (HFC-244bb) ; et
iii) la soumission de HFC-244bb à une réaction de déshydrohalogénation, pour former HFO-1234yf.

2. Méthode selon la revendication 1, dans laquelle ledit tétrachloropropène de Formule (I) est choisi dans le groupe constitué par CH₂=CClCCl₃, CCl₂=CClCH₂Cl, CHCl=CClCCl₂H, et des combinaisons de ceux-ci.

3. Méthode selon la revendication 1 ou la revendication 2, dans laquelle ladite déshydrohalogénation comprend au moins une réaction catalytique en phase gazeuse.

4. Méthode selon l'une quelconque des revendications précédentes, dans laquelle la fluoration du tétrachloropropène de Formule (I) est effectuée en utilisant du fluorure d'hydrogène en phase gazeuse.

5. Méthode selon la revendication 4, dans laquelle la fluoration du tétrachloropropène de Formule (I) est effectuée en présence d'un catalyseur.

6. Méthode selon l'une quelconque des revendications précédentes, dans laquelle la fluoration de HFO-1233xf est effectuée en phase gazeuse.

7. Méthode selon l'une quelconque des revendications 1 à 5, dans laquelle la fluoration de HFO-1233xf est effectuée en phase liquide.

8. Méthode selon la revendication 6 ou la revendication 7, dans laquelle la fluoration de HFO-1233xf est effectuée en présence d'un catalyseur.

9. Méthode selon l'une quelconque des revendications précédentes, dans laquelle la réaction de déshydro-halogénation est effectuée à une température dans la plage allant de 200°C à 800°C.

10. Méthode selon l'une quelconque des revendications précédentes, dans laquelle le tétrachloropropène de Formule (I) comprend CCl₂=CClCH₂Cl.

11. Méthode selon l'une quelconque des revendications 1 à 9, dans laquelle le tétrachloropropène de Formule (I) comprend CCl₃CCl=CH₂.
